# EUROPEAN PATENT APPLICATION

(11) **EP 2 732 846 A1**
(43) Date of publication of application: **21.05.2014**
(21) Application number: 13186793.9
(22) Date of filing: 01.10.2013
(51) Int. Cl.: A61M 25/09

(54) **Guidewire**

(30) Priority: 20.11.2012 JP 2012254154
(71) Applicant: ASAHI INTECC CO., LTD., Nagoya-shi, Aichi 463-0024 (JP)
(72) Inventor: Kosugi, Tomoki, Nagoya-shi, Aichi 463-0024 (JP); Ishihara, Kazuyuki, Nagoya-shi, Aichi 463-0024 (JP)
(74) Representative: Winter, Brandl, Fürniss, Hübner, Röss, Kaiser, Polte - Partnerschaft

(57) **Abstract**

A guidewire (10, 10a, 10b, 10c, 10d, 10e, 10f, 10g, 10h) includes a core shaft (20), an outer coil body (30) that covers an outer periphery of the core shaft (20), and an inner coil body (40, 60, 70, 80) that covers a distal portion of the core shaft (20) in the outer coil body (30). A distal end and a proximal end of the inner coil body (40, 60, 70, 80) are free ends having no bonding portions. A bonding portion for bonding the inner coil body (40, 60, 70, 80) bonds the inner coil body (40, 60, 70, 80) to only one of the core shaft (20) and the outer coil body (30).

## Description

### BACKGROUND OF THE INVENTION

### 1. Field of the Invention

The present invention relates to a medical guidewire.

### 2. Description of the Related Art

Various guidewires have been proposed which guide a catheter or the like that is inserted into a tubular organ, such as a blood vessel, an alimentary canal, or a ureter, or a body tissue for the purpose of treatment or diagnosis.

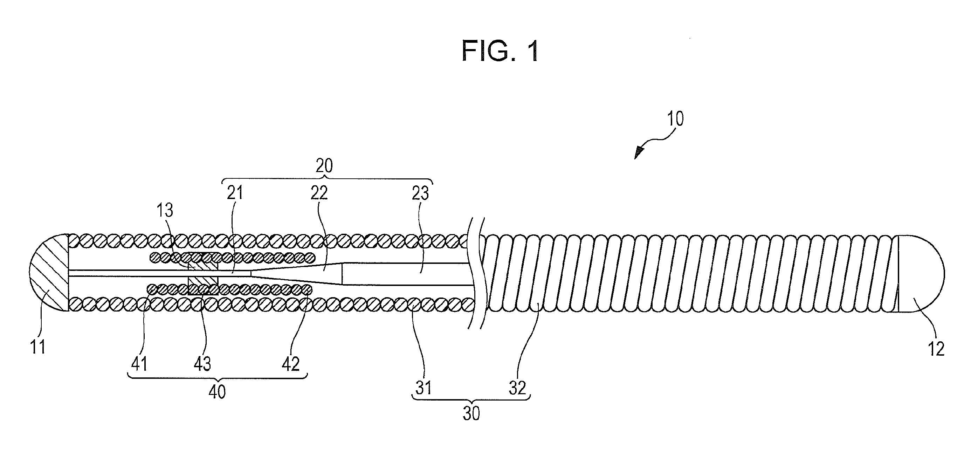

For example, Japanese Unexamined Patent Application Publication No. 8-317989 discloses a medical guidewire in which a core wire is covered with an outer coil and an inner coil made of a radiopaque material is disposed in a distal portion of the medical guidewire to increase visibility. In this medical guidewire, the distal end of the inner coil is bonded to the core wire and the outer coil by a head portion, and the proximal end of the inner coil is a free end (see, for example, Fig. 1).

However, it is difficult to make the distal portion of the medical guidewire according to Japanese Unexamined Patent Application Publication No. 8-317989 sufficiently flexible since the distal end of the inner coil is bonded to the distal ends of the core wire and the outer coil by the head portion 15.

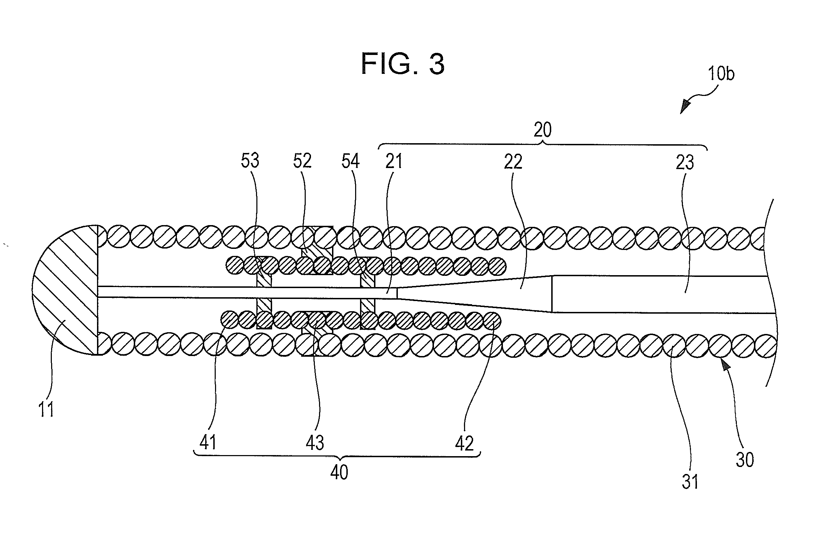

Accordingly, United States Patent No. 5,345,945 discloses a guidewire in which two coil bodies including an inner coil body and an outer coil body are provided at a distal portion of the guidewire and the inner coil body has free ends having no bonding portions so that the distal portion is sufficiently flexible (see, for example, Fig. 3).

### SUMMARY OF THE INVENTION

However, in the guidewire according to United States Patent No. 5,345,945, a bonding section for bonding the inner coil body bonds three components together, the three components being a core shaft, the inner coil body, and the outer coil body. Accordingly, the rigidity of the guidewire at the bonding section includes not only the rigidities of the core shaft, the inner coil body, and the outer coil body but also the rigidity of an inner bonding portion that bonds the inner coil body to the core shaft and the rigidity of an outer bonding portion that bonds the inner coil body to the outer coil body. In contrast, the rigidity of the guidewire at each of the free ends on the distal and proximal sides of the bonding section includes only the rigidities of the core shaft, the inner coil body, and the outer coil body. Therefore, the rigidity of the guidewire suddenly changes in the region around the bonding section, and stress concentration occurs when the guidewire is bent. As a result, plastic deformation easily occurs.

The present invention has been made in view of the above-described circumstances, and an object of the present invention is to provide a guidewire which includes a sufficiently flexible distal portion and with which plastic deformation can be prevented by suppressing stress concentration due to bending of the guidewire.

To achieve the above-described object, a guidewire according to an aspect of the present invention has the following features.

That is, according to an aspect of the present invention, a guidewire includes a core shaft, an outer coil body that covers an outer periphery of the core shaft, and an inner coil body that covers a distal portion of the core shaft in the outer coil body. A distal end and a proximal end of the inner coil body are free ends having no bonding portions. A bonding portion for bonding the inner coil body bonds the inner coil body to only one of the core shaft and the outer coil body.

In the guidewire according to the aspect, the distal and proximal ends of the inner coil body are free ends having no bonding portions, and the bonding portion for bonding the inner coil body bonds the inner coil body to only one of the core shaft and the outer coil body. Therefore, the distal portion of the guidewire is sufficiently flexible. In addition, since there is no sudden change in the rigidity of the guidewire, plastic deformation can be prevented by suppressing stress concentration due to bending of the distal portion of the guidewire.

### BRIEF DESCRIPTION OF THE DRAWINGS

Fig. 1 illustrates the overall structure of a guidewire according to a first embodiment.
Fig. 2 is an enlarged view of a part of a guidewire according to a second embodiment.
Fig. 3 is an enlarged view of a part of a guidewire according to a third embodiment.
Fig. 4 is an enlarged view of a part of a guidewire according to a fourth embodiment.
Fig. 5 is an enlarged view of a part of a guidewire according to a fifth embodiment.
Fig. 6 is an enlarged view of a part of a guidewire according to a sixth embodiment.
Fig. 7 is an enlarged view of a part of a guidewire according to a seventh embodiment.
Fig. 8 is an enlarged view of a part of a guidewire according to a first modification.
Fig. 9 is an enlarged view of a part of a guidewire according to a second modification.

### DESCRIPTION OF THE PREFERRED EMBODIMENTS

### First Embodiment

A guidewire according to a first embodiment of the present invention will be described with reference to Fig. 1. Referring to Fig. 1, a distal end of a guidewire 10 that is inserted into a body is shown at the left side, and a proximal end of the guidewire 10 that is manipulated by an operator, such as a doctor, is shown at the right side. The drawings are schematic diagrams of guidewires, and dimensional ratios of components shown in the drawings differ from the actual ratios.

As illustrated in Fig. 1, the guidewire 10 includes a core shaft 20, an outer coil body 30 that covers the outer periphery of the core shaft 20, and an inner coil body 40 that covers a distal portion of the core shaft 20 in the outer coil body 30. A distal portion of the guidewire 10 has a straight shape.

### Core Shaft

The core shaft 20 includes a small-diameter portion 21, a tapered portion 22, and a large-diameter portion 23 arranged in that order from the distal end thereof to the proximal end thereof. The small-diameter portion 21 is at the distal end of the core shaft 20, and is a most flexible portion of the core shaft 20. The small-diameter portion 21 is formed in a flat plate shape by press working. The tapered portion 22 has a circular cross section and is tapered so that the diameter thereof decreases toward the distal side. The large-diameter portion 23 has a columnar shape with a constant diameter. The arrangement and dimensions of the small-diameter portion 21, the tapered portion 22, and the large-diameter portion 23 may be changed as appropriate in order to, for example, obtain a desired rigidity. For example, the small-diameter portion 21 may have a columnar shape. The number of tapered portions and the taper angle of each tapered portion may also be set as appropriate. The material of the core shaft 20 is not particularly limited. In the present embodiment, a stainless steel (SUS304) is used. Alternatively, a superelastic alloy, such as Ni-Ti alloy, may be used as the material.

### Outer Coil Body

The outer coil body 30 includes a distal coil portion 31 and a proximal coil portion 32. The distal end of the distal coil portion 31 is bonded to the distal end of the core shaft 20 by a distal tip 11. The proximal end of the distal coil portion 31 is bonded to the distal end of the proximal coil portion 32 and the core shaft 20 by an intermediate bonding portion (not shown). The proximal end of the proximal coil portion 32 is bonded to the proximal end of the core shaft 20 by a proximal-end bonding portion 12. In the present embodiment, the distal coil portion 31 is a single-wire coil. The distal coil portion 31 has a constant outer coil diameter and a constant wire diameter over the entire length thereof. The proximal coil portion 32 is a multiple-wire coil (stranded-wire coil formed of multiple wires), and thereby has a high torque transmission performance.

### Inner Coil Body

The inner coil body 40 partially covers the small-diameter portion 21 and the tapered portion 22 of the core shaft 20. In other words, a distal end 41 of the inner coil body 40 is positioned at the small-diameter portion 21 of the core shaft 20, and a proximal end 42 of the inner coil body 40 is positioned at the tapered portion 22 of the core shaft 20. An intermediate portion 43 of the inner coil body 40 extends so as to partially cover the small-diameter portion 21 and the tapered portion 22 of the core shaft 20. The distal end 41 and the proximal end 42 of the inner coil body 40 are free ends having no bonding portions. The intermediate portion 43 of the inner coil body 40 is bonded to the small-diameter portion 21 of the core shaft 20 by an inner bonding portion 13. Thus, in the present embodiment, the inner bonding portion 13 is the only bonding portion used to bond the inner coil body 40. The inner bonding portion 13 bonds the inner coil body 40 only to the core shaft 20, and not to the outer coil body 30. The inner coil body 40 according to the present embodiment has a constant outer coil diameter and a constant wire diameter over the entire length thereof.

The material of the outer coil body 30 and the inner coil body 40 is not particularly limited. In the present embodiment, a stainless steel is used. Alternatively, a superelastic alloy, such as tungsten or Ni-Ti alloy, may be used. Alternatively, wires made of different materials may be used in combination. The material of the distal tip 11 and the bonding portions 12 and 13 is also not particularly limited. For example, a brazing material, such as an aluminum alloy brazing material, a silver brazing material, or a gold brazing material, or a metal solder, such as Au-Sn alloy, may be used.

In the guidewire 10 having the above-described structure, the distal end 41 and the proximal end 42 of the inner coil body 40 are free ends having no bonding portions. The inner bonding portion 13 for bonding the inner coil body 40 bonds the inner coil body 40 only to the core shaft 20. The rigidity variation in the distal portion of the guidewire 10 according to the present embodiment will now be discussed.

First, the rigidity variation in a region around the distal end 41 of the inner coil body 40 will be described. In a region on the distal side of the distal end 41 of the inner coil body 40, the rigidity of the guidewire 10 includes the rigidities of the "core shaft" and the "outer coil body". In a region on the proximal side of the distal end 41 of the inner coil body 40, the rigidity of the guidewire 10 includes the rigidities of the "core shaft", the "inner coil body", and the "outer coil body". Therefore, in the region around the distal end 41 of the inner coil body 40, the rigidity of the guidewire 10 changes only by the rigidity of the "inner coil body", and the rigidity variation is gradual.

Next, the rigidity variation in a region around the inner bonding portion 13 will be described. In regions on the distal side and the proximal side of the inner bonding portion 13, the rigidity of the guidewire 10 includes the rigidities of the "core shaft", the "inner coil body", and the "outer coil body". In the region where the inner bonding portion 13 is disposed, the rigidity of the guidewire 10 includes the rigidities of the "core shaft", the "inner coil body", the "outer coil body", and the "inner bonding portion". Therefore, in the region around the inner bonding portion 13, the rigidity of the guidewire 10 changes only by the rigidity of the "inner bonding portion" that bonds only the core shaft 20 and the inner coil body 40 together, and the rigidity variation is gradual.

Next, the rigidity variation in a region around the proximal end 42 of the inner coil body 40 will be described. First, in a region on the distal side of the proximal end 42 of the inner coil body 40, the rigidity of the guidewire 10 includes the rigidities of the "core shaft", the "inner coil body", and the "outer coil body". In a region on the proximal side of the proximal end 42 of the inner coil body 40, the rigidity of the guidewire 10 includes the rigidities of the "core shaft" and the "outer coil body". Therefore, in the region around the proximal end 42 of the inner coil body 40, the rigidity of the guidewire 10 changes only by the rigidity of the "inner coil body", and the rigidity variation is gradual.

As described above, in the guidewire 10 according to the present embodiment, the distal end 41 and the proximal end 42 of the inner coil body 40 are free ends having no bonding portions. The inner bonding portion 13 for bonding the inner coil body 40 bonds the inner coil body 40 to only one of the core shaft 20 and the outer coil body 30 (to the core shaft 20 in this case). Therefore, the distal portion of the guidewire is sufficiently flexible. In addition, since there is no sudden change in the rigidity of the guidewire, plastic deformation can be prevented by suppressing stress concentration due to bending of the distal portion of the guidewire.

### Second Embodiment

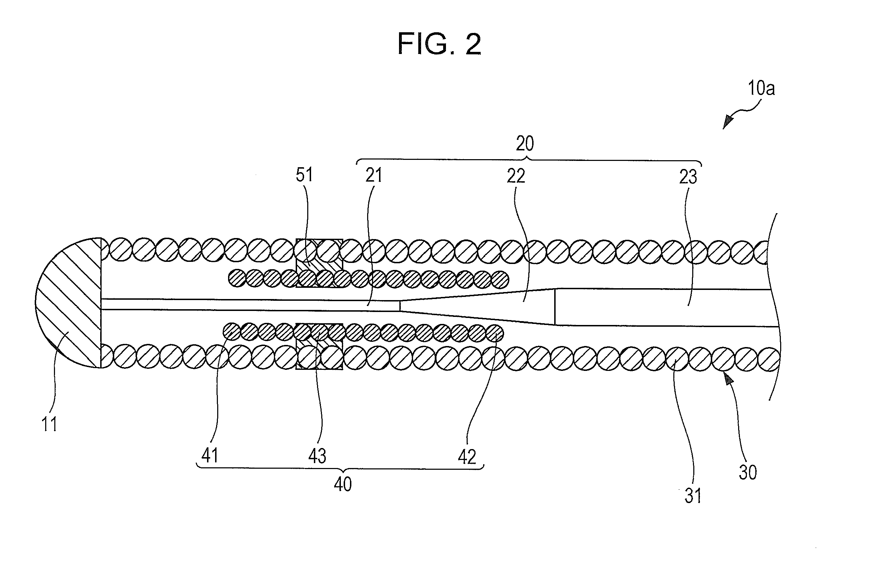

A guidewire according to a second embodiment of the present invention will be described with reference to Fig. 2. Referring to Fig. 2, a distal end of a guidewire 10a that is inserted into a body is shown at the left side, and a proximal end of the guidewire 10a that is manipulated by an operator, such as a doctor, is shown at the right side. Components similar to those in the above-described embodiment are denoted by the same reference numerals, and differences from the above-described embodiment will be mainly described.

In the guidewire 10a according to the second embodiment, the structure of a bonding portion for bonding the inner coil body 40 differs from that in the first embodiment.

As illustrated in Fig. 2, the intermediate portion 43 of the inner coil body 40 is bonded to the outer coil body 30 by an outer bonding portion 51. Thus, in the second embodiment, the outer bonding portion 51 is the only bonding portion used to bond the inner coil body 40. The outer bonding portion 51 bonds the inner coil body 40 only to the outer coil body 30, and not to the core shaft 20. Similar to the first embodiment, the distal end 41 and the proximal end 42 of the inner coil body 40 are free ends.

The rigidity variation in the distal portion of the guidewire 10a according to the second embodiment will now be discussed.

In the present embodiment, the distal end 41 and the proximal end 42 of the inner coil body 40 are free ends, as in the first embodiment. Therefore, as described above, the rigidity gradually varies in the regions around the distal end 41 and the proximal end 42 of the inner coil body 40.

Next, the rigidity variation in a region around the outer bonding portion 51 will be described. In regions on the distal side and the proximal side of the outer bonding portion 51, the rigidity of the guidewire 10a includes the rigidities of the "core shaft", the "inner coil body", and the "outer coil body". In the region where the outer bonding portion 51 is disposed, the rigidity of the guidewire 10a includes the rigidities of the "core shaft", the "inner coil body", the "outer coil body", and the "outer bonding portion". Therefore, in the region around the outer bonding portion 51, the rigidity of the guidewire 10a changes only by the rigidity of the "outer bonding portion" that bonds only the inner coil body 40 and the outer coil body 30 together, and the rigidity variation is gradual.

As described above, in the guidewire 10a according to the second embodiment, the distal end 41 and the proximal end 42 of the inner coil body 40 are free ends having no bonding portions. The outer bonding portion 51 for bonding the inner coil body 40 bonds the inner coil body 40 to only one of the core shaft 20 and the outer coil body 30 (to the outer coil body 30 in this case). Therefore, the distal portion of the guidewire is sufficiently flexible. In addition, since there is no sudden change in the rigidity of the guidewire, plastic deformation can be prevented by suppressing stress concentration due to bending of the distal portion of the guidewire.

### Third Embodiment

A guidewire according to a third embodiment of the present invention will be described with reference to Fig. 3. Referring to Fig. 3, a distal end of a guidewire 10b that is inserted into a body is shown at the left side, and a proximal end of the guidewire 10b that is manipulated by an operator, such as a doctor, is shown at the right side. Components similar to those in the above-described embodiments are denoted by the same reference numerals, and differences from the above-described embodiments will be mainly described.

In the guidewire 10b according to the third embodiment, the structure of bonding portions for bonding the inner coil body 40 differs from those in the first and second embodiments.

As illustrated in Fig. 3, the intermediate portion 43 of the inner coil body 40 is bonded to the outer coil body 30 by an outer bonding portion 52. A distal inner bonding portion 53, which bonds the inner coil body 40 to the core shaft 20, is disposed between the intermediate portion 43 and the distal end 41 of the inner coil body 40. A proximal inner bonding portion 54, which also bonds the inner coil body 40 to the core shaft 20, is disposed between the intermediate portion 43 and the proximal end 42 of the inner coil body 40. Thus, in the third embodiment, the inner coil body 40 is bonded by three bonding portions, which are the outer bonding portion 52, the distal inner bonding portion 53, and the proximal inner bonding portion 54. The outer bonding portion 52 bonds the inner coil body 40 only to the outer coil body 30, and not to the core shaft 20. The distal inner bonding portion 53 bonds the inner coil body 40 only to the core shaft 20, and not to the outer coil body 30. The proximal inner bonding portion 54 bonds the inner coil body 40 only to the core shaft 20, and not to the outer coil body 30. Similar to the first and second embodiments, the distal end 41 and the proximal end 42 of the inner coil body 40 are free ends.

The rigidity variation in the distal portion of the guidewire 10b according to the third embodiment will now be discussed.

In the present embodiment, the distal end 41 and the proximal end 42 of the inner coil body 40 are free ends, as in the first and second embodiments. Therefore, as described above, the rigidity gradually varies in the regions around the distal end 41 and the proximal end 42 of the inner coil body 40.

Next, the rigidity variation in a region around the distal inner bonding portion 53 will be described. In regions on the distal side and the proximal side of the distal inner bonding portion 53, the rigidity of the guidewire 10b includes the rigidities of the "core shaft", the "inner coil body", and the "outer coil body". In the region where the distal inner bonding portion 53 is disposed, the rigidity of the guidewire 10b includes the rigidities of the "core shaft", the "inner coil body", the "outer coil body", and the "distal inner bonding portion". Therefore, in the region around the distal inner bonding portion 53, the rigidity of the guidewire 10b changes only by the rigidity of the "distal inner bonding portion" that bonds only the core shaft 20 and the inner coil body 40 together, and the rigidity variation is gradual.

Next, the rigidity variation in a region around the outer bonding portion 52 will be described. In regions on the distal side and the proximal side of the outer bonding portion 52, the rigidity of the guidewire 10b includes the rigidities of the "core shaft", the "inner coil body", and the "outer coil body". In the region where the outer bonding portion 52 is disposed, the rigidity of the guidewire 10b includes the rigidities of the "core shaft", the "inner coil body", the "outer coil body", and the "outer bonding portion". Therefore, in the region around the outer bonding portion 52, the rigidity of the guidewire 10b changes only by the rigidity of the "outer bonding portion" that bonds only the inner coil body 40 and the outer coil body 30 together, and the rigidity variation is gradual.

Next, the rigidity variation in a region around the proximal inner bonding portion 54 will be described. In regions on the distal side and the proximal side of the proximal inner bonding portion 54, the rigidity of the guidewire 10b includes the rigidities of the "core shaft", the "inner coil body", and the "outer coil body". In the region where the proximal inner bonding portion 54 is disposed, the rigidity of the guidewire 10b includes the rigidities of the "core shaft", the "inner coil body", the "outer coil body", and the "proximal inner bonding portion". Therefore, in the region around the proximal inner bonding portion 54, the rigidity of the guidewire 10b changes only by the rigidity of the "proximal inner bonding portion" that bonds only the core shaft 20 and the inner coil body 40 together, and the rigidity variation is gradual.

As described above, in the guidewire 10b according to the third embodiment, the distal end 41 and the proximal end 42 of the inner coil body 40 are free ends having no bonding portions. The outer bonding portion 52, the distal inner bonding portion 53, and the proximal inner bonding portion 54 for bonding the inner coil body 40 each bond the inner coil body 40 to only one of the core shaft 20 and the outer coil body 30. Therefore, the distal portion of the guidewire is sufficiently flexible. In addition, since there is no sudden change in the rigidity of the guidewire, plastic deformation can be prevented by suppressing stress concentration due to bending of the distal portion of the guidewire.

### Fourth Embodiment

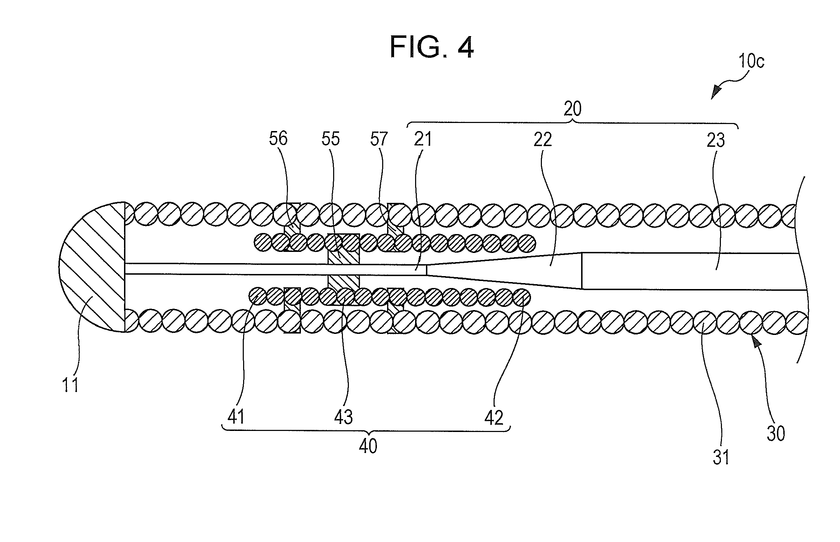

A guidewire according to a fourth embodiment of the present invention will be described with reference to Fig. 4. Referring to Fig. 4, a distal end of a guidewire 10c that is inserted into a body is shown at the left side, and a proximal end of the guidewire 10c that is manipulated by an operator, such as a doctor, is shown at the right side. Components similar to those in the above-described embodiments are denoted by the same reference numerals, and differences from the above-described embodiments will be mainly described.

In the guidewire 10c according to the fourth embodiment, the structure of bonding portions for bonding the inner coil body 40 differs from those in the first to third embodiments.

As illustrated in Fig. 4, the intermediate portion 43 of the inner coil body 40 is bonded to the core shaft 20 by an inner bonding portion 55. A distal outer bonding portion 56, which bonds the inner coil body 40 to the outer coil body 30, is disposed between the intermediate portion 43 and the distal end 41 of the inner coil body 40. A proximal outer bonding portion 57, which also bonds the inner coil body 40 to the outer coil body 30, is disposed between the intermediate portion 43 and the proximal end 42 of the inner coil body 40. Thus, in the fourth embodiment, the inner coil body 40 is bonded by three bonding portions, which are the inner bonding portion 55, the distal outer bonding portion 56, and the proximal outer bonding portion 57. The inner bonding portion 55 bonds the inner coil body 40 only to the core shaft 20, and not to the outer coil body 30. The distal outer bonding portion 56 bonds the inner coil body 40 only to the outer coil body 30, and not to the core shaft 20. The proximal outer bonding portion 57 bonds the inner coil body 40 only to the outer coil body 30, and not to the core shaft 20. Similar to the first to third embodiments, the distal end 41 and the proximal end 42 of the inner coil body 40 are free ends.

The rigidity variation in the distal portion of the guidewire 10c according to the fourth embodiment will now be discussed.

In the present embodiment, the distal end 41 and the proximal end 42 of the inner coil body 40 are free ends, as in the first to third embodiments. Therefore, as described above, the rigidity gradually varies in the regions around the distal end 41 and the proximal end 42 of the inner coil body 40.

Next, the rigidity variation in a region around the distal outer bonding portion 56 will be described. In regions on the distal side and the proximal side of the distal outer bonding portion 56, the rigidity of the guidewire 10c includes the rigidities of the "core shaft", the "inner coil body", and the "outer coil body". In the region where the distal outer bonding portion 56 is disposed, the rigidity of the guidewire 10c includes the rigidities of the "core shaft", the "inner coil body", the "outer coil body", and the "distal outer bonding portion". Therefore, in the region around the distal outer bonding portion 56, the rigidity of the guidewire 10c changes only by the rigidity of the "distal outer bonding portion" that bonds only the inner coil body 40 and the outer coil body 30 together, and the rigidity variation is gradual.

Next, the rigidity variation in a region around the inner bonding portion 55 will be described. In regions on the distal side and the proximal side of the inner bonding portion 55, the rigidity of the guidewire 10c includes the rigidities of the "core shaft", the "inner coil body", and the "outer coil body". In the region where the inner bonding portion 55 is disposed, the rigidity of the guidewire 10c includes the rigidities of the "core shaft", the "inner coil body", the "outer coil body", and the "inner bonding portion". Therefore, in the region around the inner bonding portion 55, the rigidity of the guidewire 10c changes only by the rigidity of the "inner bonding portion" that bonds only the core shaft 20 and the inner coil body 40 together, and the rigidity variation is gradual.

Next, the rigidity variation in a region around the proximal outer bonding portion 57 will be described. In regions on the distal side and the proximal side of the proximal outer bonding portion 57, the rigidity of the guidewire 10c includes the rigidities of the "core shaft", the "inner coil body", and the "outer coil body". In the region where the proximal outer bonding portion 57 is disposed, the rigidity of the guidewire 10c includes the rigidities of the "core shaft", the "inner coil body", the "outer coil body", and the "distal outer bonding portion". Therefore, in the region around the proximal outer bonding portion 57, the rigidity of the guidewire 10c changes only by the rigidity of the "proximal outer bonding portion" that bonds only the inner coil body 40 and the outer coil body 30 together, and the rigidity variation is gradual.

As described above, in the guidewire 10c according to the fourth embodiment, the distal end 41 and the proximal end 42 of the inner coil body 40 are free ends having no bonding portions. The inner bonding portion 55, the distal outer bonding portion 56, and the proximal outer bonding portion 57 for bonding the inner coil body 40 each bond the inner coil body 40 to only one of the core shaft 20 and the outer coil body 30. Therefore, the distal portion of the guidewire is sufficiently flexible. In addition, since there is no sudden change in the rigidity of the guidewire, plastic deformation can be prevented by suppressing stress concentration due to bending of the distal portion of the guidewire.

### Fifth Embodiment

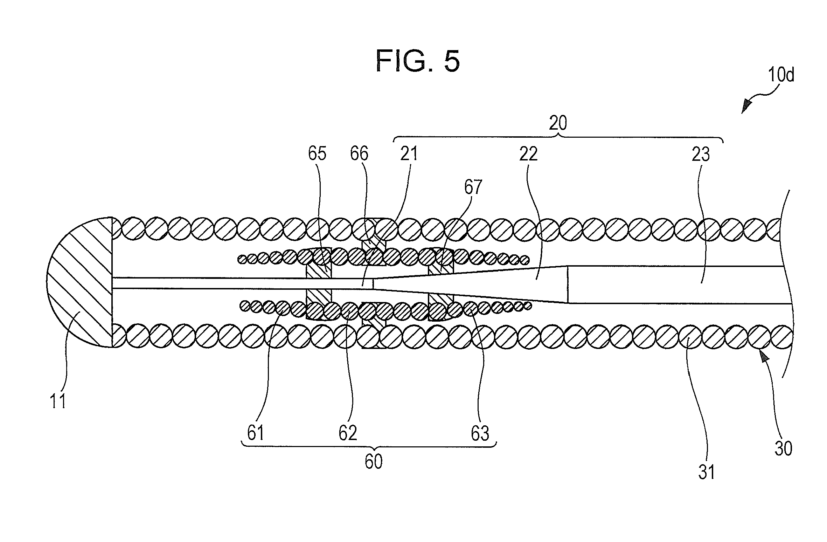

A guidewire according to a fifth embodiment of the present invention will be described with reference to Fig. 5. Referring to Fig. 5, a distal end of a guidewire 10d that is inserted into a body is shown at the left side, and a proximal end of the guidewire 10d that is manipulated by an operator, such as a doctor, is shown at the right side. Components similar to those in the above-described embodiments are denoted by the same reference numerals, and differences from the above-described embodiments will be mainly described.

The guidewire 10d according to the fifth embodiment includes an inner coil body 60 having a structure that differs from that in the first to fourth embodiments. The structure of bonding portions for bonding the inner coil body 60 is basically similar to that in the third embodiment. However, the positional relationship between the bonding portions and the core shaft differ from those in the first to fourth embodiments.

As illustrated in Fig. 5, the inner coil body 60 includes a distal coil portion 61, an intermediate coil portion 62, and a proximal coil portion 63. The coil portions 61, 62, and 63 of the inner coil body 60 are formed as an integral coil in the present invention. However, the coil portions 61, 62, and 63 may instead be formed separately. The distal end of the distal coil portion 61 is a free end having no bonding portions. The proximal end of the distal coil portion 61 is bonded to the small-diameter portion 21 of the core shaft 20 by a distal inner bonding portion 65. A central portion of the intermediate coil portion 62 is bonded to the outer coil body 30 by an outer bonding portion 66. The proximal end of the intermediate coil portion 62 is bonded to the tapered portion 22 of the core shaft 20 by a proximal inner bonding portion 67. The proximal end of the proximal coil portion 63 is a free end having no bonding portions.

In the inner coil body 60 of the present embodiment, the wire diameter and the outer coil diameter of the distal coil portion 61 decrease toward the distal end. The intermediate coil portion 62 has a constant wire diameter and a constant outer coil diameter. The wire diameter and the outer coil diameter of the proximal coil portion 63 decrease toward the proximal end. The distal coil portion 61 and the proximal coil portion 63 are processed by, for example, electropolishing. The distal coil portion 61 and the proximal coil portion 63 of the present embodiment are formed such that not only the wire diameter but also the outer coil diameter decreases. However, the distal coil portion 61 and the proximal coil portion 63 may instead be formed such that only the wire diameter decreases while the outer coil diameter is constant. Alternatively, the distal coil portion 61 and the proximal coil portion 63 may be formed such that only the outer coil diameter decreases while the wire diameter is constant. In this case, the pitch is preferably increased to ensure sufficient flexibility.

In the present embodiment, the outer bonding portion 66 is disposed at a position corresponding to the boundary between the small-diameter portion 21 and the tapered portion 22 of the core shaft 20. Accordingly, the rigidity at the proximal side of the outer bonding portion 66 is higher than that at the distal side of the outer bonding portion 66 by an amount corresponding to an increase in diameter of the tapered portion 22. In the guidewire 10d according to the present embodiment, all of the bonding portions 65 to 67 are disposed at positions where the rigidity changes. The rigidity increases in the order of the region on the distal side of the distal inner bonding portion 65, the region between the distal inner bonding portion 65 and the outer bonding portion 66, the region between the outer bonding portion 66 and the proximal inner bonding portion 67, and the region on the proximal side of the proximal inner bonding portion 67.

In the guidewire 10d according to the fifth embodiment having the above-described structure, the distal end of the distal coil portion 61 and the proximal end of the proximal coil portion 63 are free ends having no bonding portions. The distal inner bonding portion 65, the outer bonding portion 66, and the proximal inner bonding portion 67 for bonding the inner coil body 60 each bond the inner coil body 60 to only one of the core shaft 20 and the outer coil body 30. In the inner coil body 60, the outer coil diameter of the distal coil portion 61 decreases toward the distal end, and the outer coil diameter of the proximal coil portion 63 decreases toward the proximal end. Therefore, the rigidity of the guidewire 10d varies more gradually and stress concentration due to bending can be further suppressed.

In addition, in the inner coil body 60, the wire diameter of the distal coil portion 61 decreases toward the distal end, and the wire diameter of the proximal coil portion 63 decreases toward the proximal end. Therefore, the rigidity of the guidewire 10d varies more gradually and stress concentration due to bending can be further suppressed.

Since the distal coil portion 61 covers the small-diameter portion 21 of the core shaft 20, the effect of making the rigidity variation gradual is higher than that at the proximal coil portion 63 that covers the tapered portion 22, which has a diameter larger than that of the small-diameter portion 21. Therefore, the distal coil portion 61 is preferably longer than the proximal coil portion 63, and the wire diameter at the distal end of the distal coil portion 61 is preferably smaller than that at the proximal end of the proximal coil portion 63.

### Sixth Embodiment

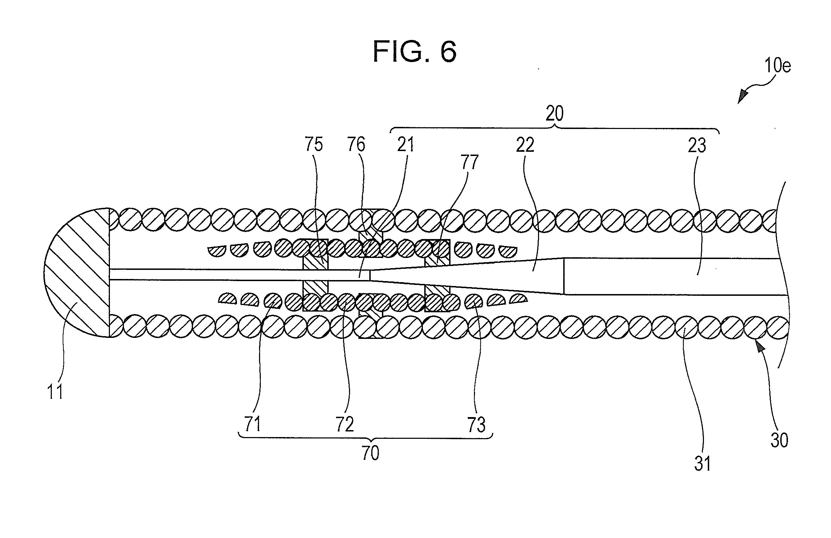

A guidewire according to a sixth embodiment of the present invention will be described with reference to Fig. 6. Referring to Fig. 6, a distal end of a guidewire 10e that is inserted into a body is shown at the left side, and a proximal end of the guidewire 10e that is manipulated by an operator, such as a doctor, is shown at the right side. Components similar to those in the above-described embodiments are denoted by the same reference numerals, and differences from the above-described embodiments will be mainly described.

The guidewire 10e according to the sixth embodiment includes an inner coil body 70 having a structure that differs from that in the first to fifth embodiments. The structure of bonding portions for bonding the inner coil body 70 is similar to that in the fifth embodiment.

As illustrated in Fig. 6, the inner coil body 70 includes a distal coil portion 71, an intermediate coil portion 72, and a proximal coil portion 73. The coil portions 71, 72, and 73 of the inner coil body 70 are formed as an integral coil in the present invention. However, the coil portions 71, 72, and 73 may instead be formed separately. The distal end of the distal coil portion 71 is a free end having no bonding portions. The proximal end of the distal coil portion 71 is bonded to the small-diameter portion 21 of the core shaft 20 by a distal inner bonding portion 75. A central portion of the intermediate coil portion 72 is bonded to the outer coil body 30 by an outer bonding portion 76. The proximal end of the intermediate coil portion 72 is bonded to the tapered portion 22 of the core shaft 20 by a proximal inner bonding portion 77. The proximal end of the proximal coil portion 73 is a free end having no bonding portions.

In the inner coil body 70 of the present embodiment, the outer peripheral surface of the distal coil portion 71 is ground and tapered so that the diameter of the distal coil portion 71 decreases toward the distal end. The intermediate coil portion 72 has a constant wire diameter and a constant outer coil diameter. The outer peripheral surface of the proximal coil portion 73 is ground and tapered so that the diameter of the proximal coil portion 73 decreases toward the proximal end. The distal coil portion 71 and the proximal coil portion 73 are processed by, for example, centerless grinding. The pitch is increased in the distal coil portion 71 and the proximal coil portion 73. By increasing the pitch, the flexibility of the inner coil body 70 can be further increased at the distal end and the proximal end. In particular, the pitch is preferably gradually increased toward the distal end and the proximal end, so that the rigidity changes gradually.

In the guidewire 10e according to the sixth embodiment having the above-described structure, the distal end of the distal coil portion 71 and the proximal end of the proximal coil portion 73 are free ends having no bonding portions. The distal inner bonding portion 75, the outer bonding portion 76, and the proximal inner bonding portion 77 for bonding the inner coil body 70 each bond the inner coil body 70 to only one of the core shaft 20 and the outer coil body 30. In the inner coil body 70, the outer coil diameter of the distal coil portion 71 decreases toward the distal end, and the outer coil diameter of the proximal coil portion 73 decreases toward the proximal end. Therefore, the rigidity of the guidewire 10e varies more gradually and stress concentration due to bending can be further suppressed.

### Seventh Embodiment

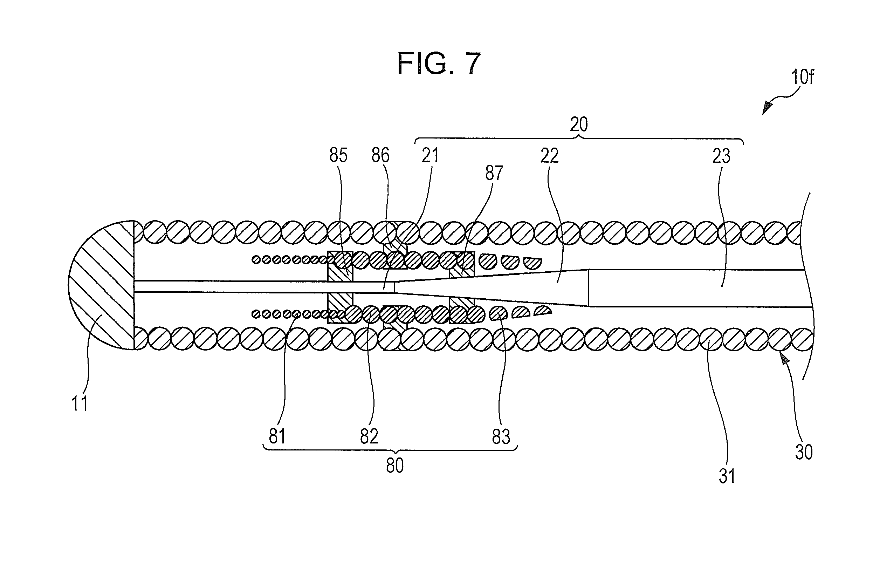

A guidewire according to a seventh embodiment of the present invention will be described with reference to Fig. 7. Referring to Fig. 7, a distal end of a guidewire 10f that is inserted into a body is shown at the left side, and a proximal end of the guidewire 10f that is manipulated by an operator, such as a doctor, is shown at the right side. Components similar to those in the above-described embodiments are denoted by the same reference numerals, and differences from the above-described embodiments will be mainly described.

The guidewire 10f according to the seventh embodiment includes an inner coil body 80 having a structure that differs from that in the first to sixth embodiments. The structure of bonding portions for bonding the inner coil body 80 is similar to that in the fifth and sixth embodiments.

As illustrated in Fig. 7, the inner coil body 80 includes a distal coil portion 81, an intermediate coil portion 82, and a proximal coil portion 83. The coil portions 81, 82, and 83 of the inner coil body 80 according to the present embodiment are formed separately. The distal end of the distal coil portion 81 is a free end having no bonding portions. The proximal end of the distal coil portion 81 is bonded to the distal end of the intermediate coil portion 82 and the small-diameter portion 21 of the core shaft 20 by a distal inner bonding portion 85. A central portion of the intermediate coil portion 82 is bonded to the outer coil body 30 by an outer bonding portion 86. The proximal end of the intermediate coil portion 82 is bonded to the distal end of the proximal coil portion 83 and the tapered portion 22 of the core shaft 20 by a proximal inner bonding portion 87. The proximal end of the proximal coil portion 83 is a free end having no bonding portions.

In the inner coil body 80 according to the present embodiment, the distal coil portion 81 has a constant wire diameter that is smaller than that of the intermediate coil portion 82. The intermediate coil portion 82 has a constant wire diameter and a constant outer coil diameter. The outer peripheral surface of the proximal coil portion 83 is ground and tapered so that the diameter of the proximal coil portion 83 decreases toward the proximal end.

In the guidewire 10f according to the seventh embodiment having the above-described structure, the inner coil body 80 includes the distal coil portion 81 which covers a flexible portion (small-diameter portion 21) of the core shaft 20 at the distal side, and the wire diameter of the distal coil portion 81 is smaller than that of the intermediate coil portion 82. Therefore, an effect of ensuring sufficient flexibility of the flexible portion (small-diameter portion 21) of the core shaft 20 while setting the rigidity of the distal portion of the guidewire 10f such that the rigidity changes stepwise can be provided in addition to the effects of the above-described embodiments. In the present embodiment, when the distal coil portion 81 of the inner coil body 80 is formed such that the wire diameter decreases toward the distal end, the rigidity of the distal portion of the guidewire 10f changes gradually.

The above-described embodiments are merely examples, and do not limit the present invention in any way. Therefore, various improvements and modifications are possible within the scope of the present invention.

For example, there may be a single bonding portion for bonding the inner coil body as described in the first and second embodiments, or there may there a plurality of bonding portions for bonding the inner coil body, e.g. three bonding portions as described in the third to sevenths embodiments. However, any bonding portion for bonding the inner coil according to the invention bonds the inner coil body to only one of the core shaft and the outer coil body. Further, the bonding portions for bonding the inner coil body are not limited to those described in the first to seventh embodiments. The bonding portions may be arranged in the longitudinal direction of a guidewire and used in combination, or be applied to a coil structure including three or more coil bodies. In the case where the bonding portions are applied to a coil structure including three or more coil bodies, bonding portions for bonding each coil body are each configured to bond the coil body to only one of another coil body and a core shaft. In addition, each inner coil is formed so as to have free ends at both ends thereof. In such a case, effects similar to those of the present invention can be obtained.

Further, irrespective of whether there is a single bonding portion for bonding the inner coil (e.g. as shown in Figs. 1 and 2) or whether there are a plurality of bonding portions for bonding the inner coil (e.g. as shown in Figs. 3 to 7) an outer coil diameter of the inner coil body at the free end at one or both of the distal end and proximal end may decrease toward the distal end and/or proximal end, respectively.

Similarly, irrespective of whether there is a single bonding portion for bonding the inner coil (e.g. as shown in Figs. 1 and 2) or whether there are a plurality of bonding portions for bonding the inner coil (e.g. as shown in Figs. 3 to 7) a wire diameter of the inner coil body at the free end at one or both of the distal end and proximal end may decrease toward the distal end and/or proximal end, respectively.

Although the inner coil bodies 40, 60, 70, and 80 partially cover the small-diameter portion 21 and the tapered portion 22 of the core shaft 20 in the first to seventh embodiments, the inner coil bodies 40, 60, 70, and 80 may instead cover only the tapered portion 22.

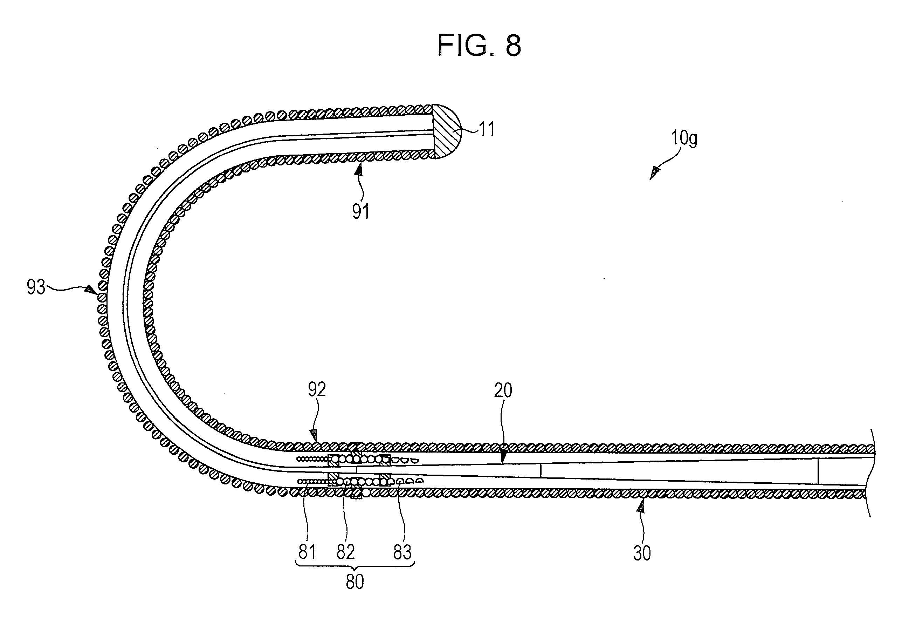

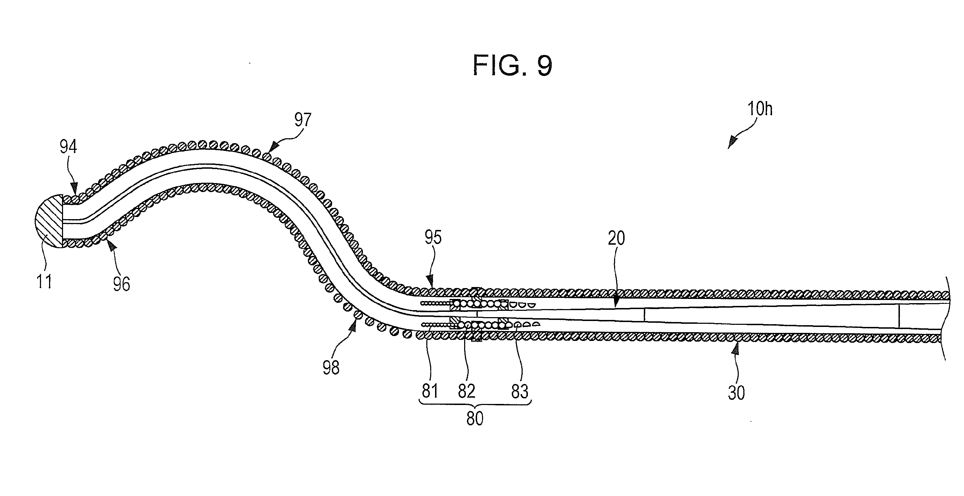

Although the distal portion of each of the guidewires 10 and 10a to 10f according to the first to seventh embodiments has a linear shape, the distal portion may instead include a curved portion. For example, Figs. 8 and 9 respectively show a first modification and a second modification of the guidewire 10f according to the seventh embodiment in which the distal portion has a curved portion.

A guidewire 10g according to the first modification illustrated in Fig. 8 includes a first linear portion 91 at the distal side, a second linear portion 92 at the proximal side, and a curved portion 93 that connects the first linear portion 91 and the second linear portion 92 to each other. The inner coil body 80 is disposed in a distal portion of the second linear portion 92. The first linear portion 91 is bent from the second linear portion 92 at the curved portion 93, so that the distal ends of the first linear portion 91 and the second linear portion 92 point in opposite directions.

The guidewire 10g according to the first modification includes the first linear portion 91 at the distal side, the second linear portion 92 at the proximal side, and the curved portion 93 that connects the first linear portion 91 and the second linear portion 92 to each other, and the inner coil body 80 is disposed in the second linear portion 92. Therefore, the inner coil body 80 serves as a flexible fulcrum at a position where the distal portion of the guidewire is bent, and blood vessel selectivity can be increased.

A guidewire 10h according to the second modification illustrated in Fig. 9 includes a first linear portion 94 at the distal side, a second linear portion 95 at the proximal side, and three curved portions 96, 97, and 98 that connect the first linear portion 94 and the second linear portion 95 to each other. The inner coil body 80 is disposed in a distal portion of the second linear portion 95. The first linear portion 94 and the second linear portion 95 are parallel to each other, and the distal ends thereof point in the same direction.

The guidewire 10h according to the second modification provides not only the effect of the above-described first modification but also an effect of increasing pushing performance in a blood vessel since the first linear portion 94 and the second linear portion 95 are parallel to each other and the distal ends thereof point in the same direction.

## Claims

1. A guidewire (10, 10a, 10b, 10c, 10d, 10e, 10f, 10g, 10h) comprising:
a core shaft (20);
an outer coil body (30) that covers an outer periphery of the core shaft (20); and
an inner coil body (40, 60, 70, 80) that covers a distal portion of the core shaft (20) in the outer coil body (30),
wherein a distal end and a proximal end of the inner coil body (40, 60, 70, 80) are free ends having no bonding portions, **characterized by**
a bonding portion for bonding the inner coil body (40, 60, 70, 80) bonds the inner coil body (40, 60, 70, 80) to only one of the core shaft (20) and the outer coil body (30).

2. The guidewire according to Claim 1, wherein an outer coil diameter of the inner coil body at the free end at one or both of the distal end and the proximal end decreases toward the distal end and/or the proximal end, respectively.

3. The guidewire according to Claim 1 or 2, wherein a wire diameter of the inner coil body at the free end at one or both of the distal end and the proximal end decreases toward the distal end and/or the proximal end, respectively.

4. The guidewire according to one of Claims 1 to 3,
wherein the core shaft (20) has a flexible portion at a distal end thereof, and
wherein the inner coil body includes a distal coil portion (61, 71, 81) that covers the flexible portion, and a wire diameter of the distal coil portion (61, 71, 81) is smaller than a wire diameter of an intermediate portion (62, 72, 82) of the inner coil body.

5. The guidewire according to one of Claims 1 to 4,
wherein each of the core shaft (20) and the outer coil body includes a first linear portion (91, 94) at a distal side, a second linear portion (92, 95) at a proximal side, and a curved portion (93, 96, 97, 98) that connects the first linear portion (91, 94) and the second linear portion (92, 95) to each other, and
wherein the inner coil body is disposed in the second linear portion (92, 95) of the outer coil body.

6. The guidewire according to Claim 5, wherein distal ends of the first linear portion (91, 94) and the second linear portion (92, 95) point in the same direction.
